# EUROPEAN PATENT APPLICATION

(11) **EP 4 198 131 A1**
(43) Date of publication of application: **21.06.2023**
(21) Application number: 21856020.9
(22) Date of filing: 19.03.2021
(51) Int. Cl.: C12N 15/77, C12N 9/10, C12N 9/80, C12P 13/00

(54) **MICROORGANISM FOR PRODUCING PUTRESCINE AND PROCESS FOR PRODUCING PUTRESCINE BY USING SAME**

(30) Priority: 13.08.2020 KR 20200101894
(71) Applicant: CJ Cheiljedang Corporation, Seoul 04560 (KR)
(72) Inventor: LEE, Jaehun, Seoul 04560 (KR); LEE, Kyoung Min, Seoul 04560 (KR); BAE, Hyun-jung, Seoul 04560 (KR)
(74) Representative: Cabinet Nony
(86) International application number: PCT/KR2021/003408
(87) International publication number: WO 2022/035011

(57) **Abstract**

The present invention relates to a microorganism for producing putrescine and a method for producing putrescine by using same.

## Description

### [Technical Field]

The present disclosure relates to a putrescine-producing microorganism and a method for producing putrescine by using same.

### [Background Art]

Putrescine, which is a raw material of nylon, is produced mainly by a chemical method using petroleum compounds as raw materials. Specifically, putrescine is produced by adding hydrogen cyanide to acrylonitrile to prepare succinonitrile, followed by hydrogenation. Such a chemical process has efficiency and economic feasibility, but has the disadvantage of being environmentally unfriendly. Hence, due to the strengthening of environmental regulations and the above, there is a need for the production of alternative substances through bio-based pathways.

In relation to this, methods for producing high concentrations of putrescine by transformation of *E*. *coli* and microorganisms of the genus *Corynebacterium* are disclosed (Morris et al., J Biol. Chem. 241: 13, 3129-3135, 1996; WO 06/005603; WO 09/125924; Qian ZD et al., Biotechnol. Bioeng. 104: 4, 651-662, 2009; Schneider et al., Appl. Microbiol. Biotechnol. 88: 4, 859-868, 2010; and Schneider et al., Appl. Microbiol. Biotechnol. 91: 17-30, 2011). Additionally, various methods for producing putrescine using microorganisms are known (US 13/992242, US 14/372000, US 14/373265, EP 2236613 B1, and US 8497098 B2).

Among the proteins associated with the putrescine biosynthesis pathway, argJ, bifunctional ornithine acetyltransferase/N-acetylglutamate synthase, is an enzyme that can perform a conversion reaction of two substances, acetyl-CoA and N-acetylornithine and has functions of both ornithine acetyltransferase (L-glutamate N-acetyltransferase) and N-acetylglutamate synthase and has both functions of an ornithine acetyltransferase and an N-acetylglutamate synthase. The enzyme argJ can reduce byproducts and lessen the burden of putrescine production since one enzyme intermediates two enzymatic reactions. However, the activity of argJ may be inhibited by intermediate metabolites, such as ornithine (Sakanyan V, Petrosyan P, Lecocq M, Boyen A, Legrain C, Demarez M, Hallet J, Glansdorff N: Genes and enzymes of the acetyl cycle of arginine biosynthesis in Corynebacterium glutamicum: enzyme evolution in the early steps of the arginine pathway. Microbiology 1996, 142:99-108), resulting in lowered biosynthesis efficiency of putrescine.

N-acetyltransferase present in *Corynebacterium glutamicum* has N-acetyl-L-glutamate producing ability in the presence of acetyl-CoA and glutamate. However, it has been reported that the N-acetyltransferase has at least 9.43 times higher specific activity than argJ (A new type of N-acetylglutamate synthase is involved in the first step of arginine biosynthesis in Corynebacterium glutamicum. "BMC genomics 2013(14) p 713).

### [Disclosure]

### [Technical Problem]

The present inventors conducted intensive efforts to increase the production of putrescine in microorganisms and, as a result, confirmed that the introduction of the activity of N-acetyltransferase derived from a strain of the genus *Corynebacterium* and the activity of acetylornithine deacetylase derived from E. *coli* into a microorganism of the genus *Corynebacterium* resulted in an increase in putrescine production, and thus completed the present disclosure.

### [Technical Solution]

In accordance with an aspect of the present disclosure, there is provided a microorganism of the genus *Corynebacterium* having putrescine producing ability, into which activity of an N-acetyltransferase derived from a strain of the genus *Corynebacterium* and activity of an acetylornithine deacetylase (argE) derived from *E. coli* are introduced.

In accordance with another aspect of the present disclosure, there is provided a method for producing putrescine, the method including culturing, in a medium, a microorganism of the genus *Corynebacterium* having putrescine producing ability, into which activity of an N-acetyltransferase derived from a strain of the genus *Corynebacterium* and activity of an acetylornithine deacetylase (argE) derived from E. *coli* are introduced.

In accordance with still another aspect of the present disclosure, there is provided a composition for producing putrescine, the composition containing a microorganism of the genus *Corynebacterium* having putrescine producing ability, into which activity of an N-acetyltransferase derived from a strain of the genus *Corynebacterium* and activity of an acetylornithine deacetylase (argE) derived from *E. coli* are introduced.

### [Advantageous Effects]

The microorganism of the genus *Corynebacterium* having putrescine producing ability, into which activity of an N-acetyltransferase derived from a strain of the genus *Corynebacterium* and activity of an acetylornithine deacetylase (argE) derived from E. *coli* are introduced, of the present disclosure, can be cultured to produce putrescine with a high yield compared to an existing non-modified microorganism.

### [Brief Description of Drawings]

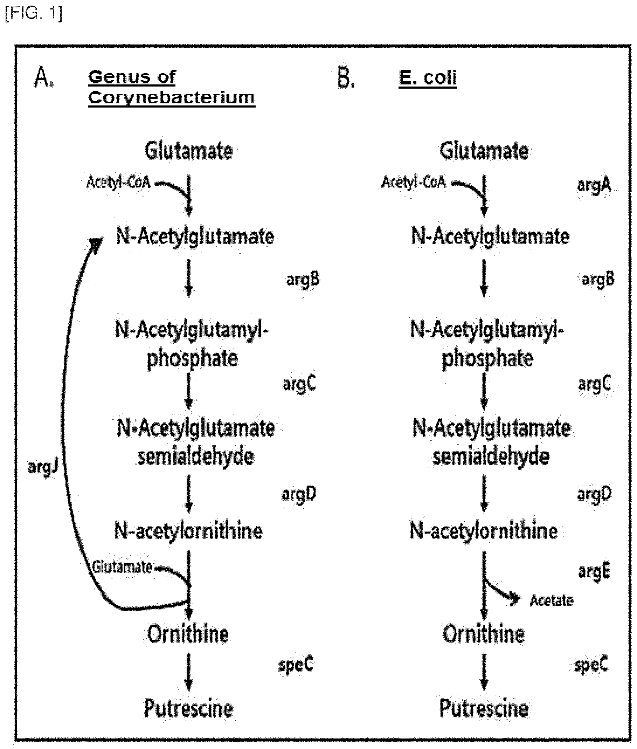

FIG. 1 schematically shows the putrescine and ornithine biosynthesis pathway in a microorganism of the genus *Corynebacterium* (A) and the putrescine and ornithine biosynthesis pathway in E. *coli* (B).

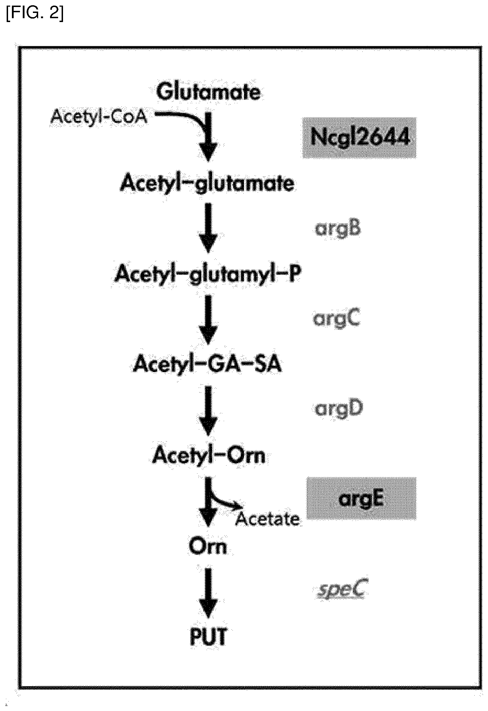

FIG. 2 schematically shows the putrescine and ornithine biosynthesis pathway in a microorganism of the genus *Corynebacterium* with improved putrescine and ornithine producing ability through *Coryne* Ncgl2644 enhancement and E. *coli* argE introduction.

### [Detailed Description of the Invention]

The present disclosure will be specifically described as follows. Each description and embodiment in the present disclosure may also be applied to other descriptions and embodiments. That is, all combinations of various elements in the present disclosure fall within the scope of the present disclosure. Furthermore, the scope of the present disclosure is not limited by the specific description below.

In accordance with an aspect of the present disclosure, there is provided a microorganism of the genus *Corynebacterium* having putrescine producing ability, into which activity of an N-acetyltransferase derived from a strain of the genus *Corynebacterium* and activity of an acetylornithine deacetylase (argE) derived from *E. coli* are introduced.

As used herein, the term "putrescine" refers to a diamine organic compound composed of four carbon atoms which has the molecular formula NH₂(CH₂)₄NH₂ and also named 1,4-diaminobutane or butanediamine.
As used herein, the term "N-acetyltransferase" refers to an enzyme (EC 2.3.1.5) that catalyzes the transfer of an acetyl group from acetyl-CoA to arylamine, arylhydroxylamine, and arylhydrazine. The enzyme can catalyze acetyl transfer between arylamines without CoA and can have N-acetyl-L-glutamate producing ability in the presence of acetyl-CoA and glutamate. The enzyme is known to have wide specificity for aromatic amines.

In the present disclosure, the N-acetyltransferase may belong to a family of GCN5-related N-acetyltransferases (GNAT), but is not limited thereto.

The N-acetyltransferase may have the amino acid sequence of SEQ ID NO: 1, consist of the amino acid sequence of SEQ ID NO: 1, or contain the amino acid sequence as set forth in SEQ ID NO: 1, but is not limited thereto. The sequence of SEQ ID NO: 1 may be confirmed in the known database NCBI GenBank.

Specifically, the N-acetyltransferase may have the amino acid sequence of SEQ ID NO 1 and/or an amino acid sequence having at least 70%, 80%, 85%, 90%, 95%, 96%, 97%, 98%, or 99% or more homology or identity with SEQ ID NO: 1. It is also obvious that even N-acetyltransferase having an amino acid sequence with deletion, modification, substitution, or addition in a part thereof may fall within the scope of the present disclosure as long as the amino acid sequence has such homology or identity and exhibits a function corresponding to the N-acetyltransferase. That is, although described as "protein or polypeptide containing the amino acid sequence as set forth in a specific sequence number" or "protein or polypeptide consisting of the amino acid sequence as set forth in a specific sequence number" in the present disclosure, any protein consisting of an amino acid sequence with deletion, modification, substitution, or addition in a part thereof may also be used in the present disclosure as long as the protein has the identical or corresponding activity identical or corresponding to that of the polypeptide consisting of the amino acid sequence of the corresponding sequence number. For example, it is obvious that any polypeptide that has the identical or corresponding activity to "polypeptide containing the amino acid sequence of SEQ ID NO: 1" may fall within "polypeptide containing the amino acid sequence of SEQ ID NO: 1".

In the present disclosure, the gene encoding the N-acetyltransferase may be derived from a strain of the genus *Corynebacterium,* and may be specifically derived from *Corynebacterium glutamicum,* but any strain of the genus *Corynebacterium* that can express the gene encoding N-acetyltransferase is not particularly limited. The gene may contain a nucleotide sequence encoding the amino acid sequence of SEQ ID NO: 1, and more specifically, may contain the nucleotide sequence of SEQ ID NO: 2, but is not limited thereto. The nucleotide sequence of SEQ ID NO: 2 may be obtained from the known database GenBank and named Ncgl2644. The gene containing the nucleotide sequence of SEQ ID NO: 2 may be used interchangeably with a polynucleotide containing the nucleotide sequence of SEQ ID NO: 2, a gene or polynucleotide having the nucleotide sequence of SEQ ID NO: 2, or a gene or polynucleotide consisting of the nucleotide sequence of SEQ ID NO: 2.

As used herein, the term "polynucleotide" refers to a polymer of nucleotides chain-extended lengthwise by a covalent bond of nucleotide monomers, and in general a DNA or RNA strand with a certain length, and more specifically, may mean a polynucleotide fragment encoding the variant. The polynucleotide may be described as a gene when the polynucleotide is an assemblage of polynucleotides capable of carrying out functions. In the present disclosure, polynucleotides and genes may be used interchangeably,

Specifically, due to codon degeneracy or by considering codons preferred by a microorganism in which the polypeptide is allowed to express, the polynucleotide of the present disclosure may be variously modified in a coding region thereof within the range in which the amino acid sequence of the polypeptide is not changed. Specifically, any polynucleotide sequence that encodes N-acetyltransferase consisting of the amino acid sequence of SEQ ID NO: 1 may be included without limitation.

In addition, any probe that can be prepared from a known gene sequence, for example, any sequence that can hybridize with a complementary sequence to a part or the entirety of the nucleotide sequence under stringent conditions to encode the N-acetyltransferase consisting of the amino acid sequence of SEQ ID NO: 1 may be included without limitation. The term "stringent conditions" refers to conditions that enable specific hybridization between polynucleotides. Such conditions are well known in the art. For example, the conditions may include conditions under which genes having high homology or identity, such as, genes having at least 40%, specifically at least 90%, more specifically at least 95%, still more specifically at least 97%, and still more specifically at least 99% homology or identity hybridize with each other but genes having lower homology or identity do not hybridize with each other; or typical washing conditions for southern hybridization, i.e., conditions where washing is conducted once, specifically, twice or three times at a salt concentration and temperature corresponding to 60°C, 1XSSC, and 0.1% SDS, specifically 60°C, 0.1XSSC, and 0.1% SDS, and more specifically 68°C, 0.1XSSC, and 0.1 % SDS.

The hybridization requires that two nucleic acids have complementary sequences although there may be mismatches between nucleotides according to the stringency of hybridization. The term "complementary" is used to describe the relationship between nucleotide bases that can hybridize with each another. For example, as for DNA, adenosine is complementary to thymine, and cytosine is complementary to guanine. Therefore, the present disclosure may include not only substantially similar nucleic acid sequences but also isolated nucleic acid fragments complementary to the entire sequence.

Specifically, polynucleotides having homology or identity may be detected using the above-described hybridization conditions including a hybridization step at a Tm value of 55°C. In addition, the Tm value may be 60°C, 63°C, or 65°C, but is not limited thereto, and may be appropriately adjusted by a person skilled in the art according to the purpose.

An appropriate degree of stringency for hybridization of polynucleotides may depend on lengths and the degree of complementarity of the polynucleotides, and parameters thereof are well known in the art (see Sambrook et al., supra, 9.50-9.51, 11.7-11.8).

As used herein, the term "homology" or "identity" refers to the degree of similarity between two given amino acid sequences or nucleotide sequences and may be expressed as a percentage. The terms homology and identity may be often used interchangeably.

The sequence homology or identity of conserved polynucleotides or polypeptides may be determined by standard alignment algorithms, and default gap penalties established by a program to be used may be used together. Substantially, homologous or identical sequences may generally hybridize with each other as a whole or in part under moderate or highly stringent conditions. It is obvious that the hybridization also includes hybridization with a polynucleotide containing usual codons or codons considering codon degeneracy in the polynucleotide.

The sequence homology, similarity, or identity between two polynucleotides or polypeptide sequences may be determined using any computer algorithm known in the art, such as the "FASTA" program, using default parameters disclosed by Pearson et al (1988) [Proc. Natl. Acad. Sci. USA 85]: 2444. Alternatively, the homology, similarity, or identity may be determined using the Needleman-Wunsch algorithm (Needleman and Wunsch, 1970, J. Mol. Biol. 48: 443-453) as implemented in the Needleman program of the European Molecular Biology Open Software Suite (EMBOSS) package (Rice et al., 2000, Trends Genet. 16: 276-277) (version 5.0.0 or later) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.] (1988) SIAM J Applied Math 48: 1073). For example, the homology, similarity, or identity may be determined using BLAST of the National Center for Biotechnology Information database, or ClustalW.

The homology, similarity, or identity between polynucleotides or polypeptides may be determined by comparing sequence information using the GAP computer program, for example, Needleman et al., (1970), J Mol Biol. 48:443, as known in Smith and Waterman, Adv. Appl. Math (1981) 2:482. Briefly, the GAP program defines similarity as the number of aligned symbols (i.e., nucleotides or amino acids) which are similar, divided by the total number of symbols in a shorter of two sequences. Default parameters for the GAP program may include: (1) a binary comparison matrix (containing a value of 1 for identity and 0 for non-identity) and a weighted comparison matrix of Gribskov et al. (1986) Nucl. Acids Res. 14:6745 as disclosed in Schwartz and Dayhoff, eds., Atlas Of Protein Sequence And Structure, National Biomedical Research Foundation, pp. 353-358 (1979) (or the EDNAFULL (EMBOSS version of NCBI NUC4.4) substitution matrix); (2) a penalty of 3.0 for each gap and an additional 0.10 penalty for each symbol in each gap (or a gap open penalty of 10, and a gap extension penalty of 0.5); and (3) no penalty for end gaps.

In an embodiment of the present disclosure, the variant of the present disclosure may have phytoene desaturase activity. In addition, the variant of the present disclosure may have an activity to increase 5'-inosine monophosphate (IMP) producing ability compared with a wild-type polypeptide having phytoene desaturase activity.

As used herein, the term "phytoene desaturase" is a polypeptide that converts colorless 15-cis-phytoene to bright red lycopene in a biochemical pathway called a poly-trans pathway. Specifically, the phytoene desaturase of the present disclosure may be used interchangeably with phytoene desaturase or Crtl. In the present disclosure, the sequence of the phytoene desaturase may be obtained from the known database NCBI GenBank. Specifically, the phytoene desaturase may be a polypeptide having phytoene desaturase encoded by *crtl,* but is not limited thereto.

As used herein, the term "acetylornithine deacetylase" refers to an enzyme (EC 3.5.1.16) that mediates the reaction involved in the production of acetic acid and ornithine by mediating the hydrolysis of acetylornithine. In the present disclosure, the acetylornithine deacetylase may be argE, but is not limited thereto.

The argE may have the amino acid sequence of SEQ ID NO: 3, consist of the amino acid sequence of SEQ ID NO: 3, or contain the amino acid sequence as set forth in SEQ ID NO: 3, but is not limited thereto. The sequence of SEQ ID NO: 3 may be confirmed in the known database NCBI GenBank.

In the present disclosure, the gene encoding argE may be derived from bacteria, and specifically, may be derived from *E. coli,* but any strain that can express the gene encoding argE is not particularly limited. The gene may contain a nucleotide sequence encoding the amino acid sequence of SEQ ID NO: 3, and more specifically, may contain the nucleotide sequence of SEQ ID NO: 4, but is not limited thereto. The nucleotide sequence of SEQ ID NO: 4 may be obtained from the known database GenBank.

In the present disclosure, the microorganism of the genus *Corynebacterium* having putrescine producing ability may have weakened activity of bifunctional ornithine acetyltransferase/N-acetylglutamate synthase (argJ) derived from a strain of the genus *Corynebacterium,* but is not limited thereto.

As used herein, the term "bifunctional ornithine acetyltransferase/N-acetylglutamate synthase" refers to an enzyme that can perform conversion reactions of two substances, acetyl-CoA and N-acetylornithine, and the enzyme has functions of both ornithine acetyltransferase (L-glutamate N-acetyltransferase (EC 2.3.1.35)) and N-acetylglutamate synthase (EC 2.3.1.1). The ornithine acetyltransferase converts N2-acetyl-L-ornithine and L-glutamate into L-ornithine, and the N-acetyl glutamate synthetase catalyzes the production of N-acetyl glutamate from glutamate and acetyl-CoA. In the present disclosure, the bifunctional ornithine acetyltransferase/N-acetylglutamate synthase may be argJ, but is not limited thereto.

The argJ can reduce byproducts and lessen the burden of putrescine production since one enzyme intermediate two enzymatic reactions. However, the activity of argJ may be inhibited by metabolic intermediates, such as ornithine, resulting in lowered biosynthesis efficiency of putrescine.

The argJ may have the amino acid sequence of SEQ ID NO: 5 or SEQ ID NO: 7, consist of the amino acid sequence of SEQ ID NO: 5 or SEQ ID NO: 7, or contain the amino acid sequence AS set forth in SEQ ID NO: 5 or SEQ ID NO: 7, but is not limited thereto. The sequence of SEQ ID NO: 5 or SEQ ID NO: 7 may be confirmed in the known database NCBI GenBank.

In the present disclosure, the gene encoding argJ may be derived from a strain of the genus *Corynebacterium* and, specifically, may be derived from *Corynebacterium glutamicum,* but any strain of the genus *Corynebacterium* that can express the gene encoding argJ is not particularly limited. Specifically, the gene encoding the amino acid sequence of SEQ ID NO: 5 may be derived from *Corynebacterium glutamicum* ATCC13869, but is not limited thereto. The gene may contain a nucleotide sequence encoding the amino acid sequence of SEQ ID NO: 5, and more specifically, may contain the nucleotide sequence of SEQ ID NO: 6, but is not limited thereto. The nucleotide sequence of SEQ ID NO: 6 may be obtained from the known database GenBank.

In addition, the gene encoding the amino acid sequence of SEQ ID NO: 7 may be derived from *Corynebacterium glutamicum* ATCC13032, but is not limited thereto. The gene may contain a nucleotide sequence encoding the amino acid sequence of SEQ ID NO: 7, and more specifically, may contain the nucleotide sequence of SEQ ID NO: 8, but is not limited thereto. The nucleotide sequence of SEQ ID NO: 8 may be obtained from the known database GenBank.

As used herein, the term "microorganism having putrescine producing ability" refers to a microorganism naturally having putrescine producing ability or a microorganism obtained by imparting putrescine producing ability to a parent strain having no putrescine producing ability. Specifically, the microorganism is a microorganism producing putrescine, into which activity of N-acetyltransferase derived from a strain of the genus *Corynebacterium* and activity of argE derived from *E. coli* are introduced, but is not limited thereto.

Specifically, the "microorganism producing putrescine" includes all of wild-type microorganisms or naturally or artificially genetically modified microorganisms. More specifically, the microorganism producing putrescine may be a microorganism in which a specific mechanism is weakened or enhanced due to the insertion of an exogenous gene or the enhancement or weakening of activity of an intrinsic gene, wherein the microorganism may have genetic mutation or enhanced putrescine producing activity for the production of target putrescine.

Specifically, the term "introduction" of activity means that a gene not originally possessed by a microorganism is expressed in the microorganism and thus the microorganism exhibits the activity of a specific protein or exhibits activity of the corresponding protein increased or enhanced compared with the intrinsic activity or the activity before modification. For example, the term may indicate that a polynucleotide encoding a specific protein is introduced into the chromosome of a microorganism or a vector containing a polynucleotide encoding a specific protein is introduced into a microorganism, thereby exhibiting the activity of the specific protein.

As used herein, the term "enhancement" in activity of polypeptide activity means that the activity of a polypeptide is increased compared with the intrinsic activity. The enhancement may be used interchangeably with terms such as activation, up-regulation, overexpression, and increase. In particular, activation, enhancement, up-regulation, overexpression, and increase may include both exhibiting activity that was not originally possessed or exhibiting improved activity compared with the intrinsic activity or activity before modification. The "intrinsic activity" means the activity of a specific polypeptide originally possessed by a parent strain before trait change or a non-modified microorganism when a trait is changed by genetic variation due to natural or artificial factors. This term may be used interchangeably with the "activity before modification". The fact that the activity of a polypeptide is "enhanced", "up-regulated", "overexpressed", or "increased" compared with the intrinsic activity means that the activity of a polypeptide is improved compared with the activity and/or concentration (expression level) of a specific polypeptide originally possessed by a parent strain before the trait is changed or a non-modified microorganism.

The enhancement may be achieved through the introduction of an exogenous polypeptide or the enhancement of activity and/or concentration (expression level) of an endogenous polypeptide. The enhancement of the activity of a polypeptide may be checked by an increase in the degree of activity or expression level of the corresponding polypeptide or an increase in the amount of a product produced from the corresponding polypeptide.

For the enhancement of the activity of a polypeptide, various methods well known in the art may be applied, and the method is not limited as long as the activity of a target polypeptide can be enhanced compared with a microorganism before modification. Specifically, genetic engineering and/or protein engineering well known to those skilled in the art, which are routine methods of molecular biology, may be used, but the method is not limited thereto (for example, Sitnicka et al., Functional Analysis of Genes. Advances in Cell Biology. 2010, Vol. 2. 1-16; Sambrook et al., Molecular Cloning 2012; and the like).

Specifically, the enhancement of a polypeptide of the present disclosure may indicate:
1) increase in the intracellular copy number of a polynucleotide encoding the polypeptide;
2) replacement of a gene expression control region on the chromosome encoding the polypeptide with a sequence having strong activity;
3) modification of a nucleotide sequence encoding a start codon or 5'-UTR region of the gene transcript encoding the polypeptide;
4) modification of the amino acid sequence of the polypeptide to enhance activity of the polypeptide;
5) modification of a polynucleotide sequence encoding the polypeptide to enhance the activity of the polypeptide (e.g., modification of a polynucleotide sequence of the polypeptide gene to encode a polypeptide modified to enhance activity of the polypeptide);
6) introduction of an exogenous polypeptide exhibiting activity of the polypeptide or an exogenous polynucleotide encoding the polypeptide;
7) codon optimization of a polynucleotide encoding the polypeptide;
8) modification or chemical modification of an exposed region selected by analysis of the tertiary structure of the polypeptide; or
9) a combination of two or more selected from items 1) to 8), but is not particularly limited thereto.

More specifically, the above items are described as follows.

The increase in the intracellular copy number of a polynucleotide encoding the polypeptide in item 1) above may be attained by the introduction, into a host cell, of a vector to which the polynucleotide encoding the corresponding polypeptide is operatively linked and which can replicate and function independently of the host. Alternatively, the increase may be attained by the introduction of one or more copies of the polynucleotide encoding the corresponding polypeptide into the chromosome in a host cell. The introduction into the chromosome may be performed by the introduction, into a host cell, of a vector capable of inserting the polynucleotide into the chromosome in the host cell, but is not limited thereto. The vector is as described above.

The replacement of a gene expression control region on the chromosome encoding the polypeptide with a sequence with strong activity in item 2) may be, for example, the occurrence of modification on the sequence through deletion, insertion, non-conservative or conservative substitution, or a combination thereof, or the replacement with a sequence having stronger activity, so as to further enhance the activity of the expression control region. The expression control region may include, but not particularly limited to, a promoter, an operator sequence, a sequence encoding a ribosome binding site, a sequence controlling the termination of transcription and translation, and the like. For example, the replacement may be to replace the original promoter with a strong promoter, but is not limited thereto.

Examples of the known strong promoter may include CJ1 to CJ7 promoters (US 7662943 B2), lac promoter, trp promoter, trc promoter, tac promoter, lamda phage PR promoter, PL promoter, tet promoter, gapA promoter, SPL7 promoter, SPL13 (sm3) promoter (US 10584338 B2), O2 promoter (US 10273491 B2), tkt promoter, yccA promoter, and the like, but are not limited thereto.

The modification of a nucleotide sequence encoding a start codon or 5'-UTR region of the gene transcript encoding the polypeptide in item 3) may be, for example, the substitution with a nucleotide sequence encoding, rather than an endogenous start codon, another start codon having a higher expression rate of a polypeptide, but is not limited thereto.

The modification of the amino acid sequence or the polynucleotide sequence in items 4) and 5) may be the occurrence of modification on the sequence through deletion, insertion, non-conservative or conservative substitution, or a combination thereof in the amino acid sequence of the polypeptide or the polynucleotide sequence encoding the polypeptide, or the replacement with an amino acid sequence or polynucleotide sequence modified to have stronger activity or an amino acid sequence or polynucleotide sequence modified to have increased activity, so as to enhance activity of the polypeptide, but is not limited thereto. Specifically, the replacement may be performed by inserting the polynucleotide into the chromosome by homologous recombination, but is not limited thereto. The vector used may further include a selection maker for checking the insertion of the chromosome. The selection marker is as described above.

The introduction of an exogenous polypeptide exhibiting activity of the polypeptide in item 6) may be the introduction, into a host cell, of an exogenous polynucleotide encoding a polypeptide exhibiting the same/similar activity to the polypeptide. The exogenous polynucleotide is not limited to the origin or sequence thereof as long as the exogenous polynucleotide exhibits the identical /similar activity with regard to the polynucleotide. The introduction may be performed by any known transformation method appropriately selected by a person skilled in the art, and the introduced polynucleotide is expressed in the host cell, and thus the polypeptide is produced and the activity thereof can be enhanced.

The codon optimization of a polynucleotide encoding the polypeptide in item 7) may be the codon optimization of an endogenous polynucleotide so as to increase transcription or translation thereof in a host cell, or the codon optimization of an exogenous polynucleotide so as to perform optimized transcription or translation in a host cell.

The modification or chemical modification of an exposed region selected by analysis of the tertiary structure of the polypeptide in item 8) may be, for example, the modification or chemical modification of an exposed region to be modified or chemically modified, by comparing sequence information of a polypeptide to be analyzed with a database that stores sequence information of known proteins to determine a template protein candidate according to similarity of the sequences and identifying the structure on the basis of the determined candidate.

Such an enhancement of the activity of the polypeptide may mean that the activity or concentration or expression level of the corresponding polypeptide is increased relative to the activity or concentration of the polypeptide expressed in a wild type microbial strain or a microbial strain before modification, or that the amount of a product produced from the corresponding polypeptide is increased, but is not limited thereto.

The modification of a part or the entirety of the polynucleotide in the microorganism of the present disclosure may be induced by (a) homologous recombination using a vector for chromosome insertion in the microorganism or genome editing using engineered nuclease (e.g., CRISPR-Cas9) and/or (b) treatment with light, such as ultraviolet light and radiation, and/or chemicals, but is not limited thereto. The method of modifying a part or the entirety of the gene may include a method by DNA recombinant technology. For example, by introducing a nucleotide sequence or vector, containing a nucleotide sequence homologous to a target gene, into the microorganism to cause homologous recombination, a part or the entirety of the gene may be deleted. The introduced nucleotide sequence or vector may contain a dominant selection marker, but is not limited thereto.

Such an enhancement of the activity of a protein may mean that the activity or concentration of the corresponding protein is increased compared with the activity or concentration of the protein expressed in a wild-type microbial strain or a microbial strain before modification, but is not limited thereto. As used herein, the term "strain before modification" or "microorganism before modification" does not exclude a strain containing mutation that may naturally occur in the microorganism, and refers to a native strain itself or a strain before a trait is changed due to genetic mutation caused by artificial factors. In the present disclosure, the trait change may be an introduction of activity of N-acetyltransferase derived from a strain of the genus *Corynebacterium* and activity of argE derived from *E. coli.* The "strain before modification" or "microorganism before modification" may be used interchangeably with "non-mutated strain", "non-modified strain", "non-mutated microorganism", "non-modified microorganism", or "reference microorganism".

In the present disclosure, the reference microorganism is not particularly limited as long as the reference microorganism produces putrescine, and a mutated strain having enhanced putrescine producing ability compared with a wild-type microorganism is also included without limitation. Examples thereof may include wild-type *Corynebacterium glutamicum* ATCC13032, the wild-type strain *Corynebacterium glutamicum* ATCC13869, or strains in which one or more genetic modifications are added to the above strains so as to enhance the putrescine biosynthesis pathway, but are not limited thereto.

The one or more genetic modifications may be, for example, any one or more genetic modifications selected from: enhancing a gene in the putrescine biosynthesis pathway; overexpressing the activity of the putrescine operon; improving the supply and efficiency of a precursor of putrescine; improving the export of putrescine; and weakening the activity of a gene in a competitive pathway, a regulator in the directional pathway of the putrescine operon, or a putrescine importer gene, and putrescine importer and lysis genes, but are not limited thereto.

The genetic modification of enhancing a gene in the putrescine biosynthesis pathway may be, for example, an introduction of the gene (speC) encoding ornithine decarboxylase (ODC) derived from wild-type *E. coli* W3110 into the chromosome, but is not limited thereto.

The genetic modification of overexpressing the activity of the putrescine operon may be, for example, a substitution of a promoter of the argCJBD gene cluster encoding enzymes involved in the synthesis of ornithine from glutamate, but is not limited thereto.

The genetic modification of improving the export of putrescine and weakening the activity of a gene in a competitive pathway, a regulator in the directional pathway of the putrescine operon, or a putrescine importer gene, and putrescine importer and lysis genes may be, for example, a deletion of the gene (argF) encoding ornithine carbamoyl transferase and the gene (NCgl1221) encoding the glutamate exporter involved in glutamate export in the chromosome; or an inactivation of the activity of NCgl1469 protein defined as histone acetyltransferase HPA2 and related acetyltransferase, but is not limited thereto.

A strain having one or more of the genetic modifications may be, for example, CC01-0064 strain having putrescine producing ability in which: in ATCC13032, the gene (argF) encoding ornithine carbamoyl transferase and the gene (NCgl1221) encoding the glutamate exporter involved in glutamate export in the chromosome are deleted; the gene (speC) encoding ornithine decarboxylase (ODC) derived from wild-type *E. coli* W3110 is introduced into the chromosome; and a promoter of the argCJBD gene cluster encoding enzymes involved in the synthesis of ornithine from glutamate is substituted (KCCM11138P, Korean Patent Publication No. 2012-0064046) or CC01-0063 strain in which the activity of NCgl1469 protein defined as histone acetyltransferase HPA2 and related acetyltransferase is inactivated in the CC01-0064 strain (KCCM11240P, Korean Patent Publication No. 10-2013-0082478), but is not limited thereto.

Alternatively, the microorganism producing putrescine may have weakened activity of argJ derived from a strain of the genus *Corynebacterium,* but is not limited thereto.

As used herein, the term "weakening" of the protein has a concept encompassing all of the reduction of activity or the absence of activity compared with the intrinsic activity. The weakening may be used interchangeably with inactivation, deficiency, down-regulation, decrease, reduction, attenuation, or the like.

The weakening may also include: a case where the activity of the polypeptide itself is reduced or eliminated compared with the activity of the polypeptide possessed by the original microorganism due to mutation or the like of a polynucleotide encoding the polypeptide; a case where the entire activity and/or concentration (expression level) of the polypeptide in a cell is lower compared with the native strain due to the inhibition of the expression of a gene of a polynucleotide encoding the polypeptide or the inhibition of the translation into the polypeptide; a case where the expression of the polynucleotide is not attained; and/or a case where the polypeptide has no activity in spite of the expression of the polynucleotide. The "intrinsic activity" refers to the activity of a specific polypeptide originally possessed by a parent strain before modification or a wild-type or non-modified microorganism when a trait is changed due to genetic mutation caused by natural or artificial factors. This term may be used interchangeably with the "activity before modification". The "inactivation, deficiency, decrease, down-regulation, reduction, or attenuation" of the activity of a polypeptide compared with the intrinsic activity thereof means that the activity of the polypeptide is lowered compared with the activity of a specific polypeptide originally possessed by a parent strain before transformation or a non-modified microorganism.

The weakening of the activity of the polypeptide may be performing by any method known in the art, but is not limited thereto, and may be attained by applying various methods well known in the art (e.g., Nakashima N et al., Bacterial cellular engineering by genome editing and gene silencing. Int J Mol Sci. 2014;15(2):2773-2793; and Sambrook et al. Molecular Cloning 2012, etc.).

In the present disclosure, a protein to be a target of weakening, i.e., a target protein, may be argJ, but is not limited thereto.
1) deletion of a part or the entirety of the gene encoding the polypeptide;
2) modification of an expression control region (or expression control sequence) so as to reduce the expression of the gene encoding the polypeptide;
3) modification of the amino acid sequence (e.g., deletion/substitution/addition of at least one amino acid in the amino acid sequence) constituting the polypeptide so as to eliminate or weaken the activity of the polypeptide;
4) modification of the gene sequence encoding the polynucleotide to eliminate or weaken the activity of the polypeptide (e.g., deletion/substitution/addition of at least one nucleic acid nucleotide in the nucleic acid nucleotide sequence of the polypeptide gene so as to encode the polypeptide modified to eliminate or weaken the activity of the polypeptide);
5) modification of a nucleotide sequence encoding a start codon or 5'-UTR region of the gene transcript encoding the polypeptide;
6) introduction of an antisense oligonucleotide (e.g., antisense RNA) complementarily binding to the gene transcript encoding the polypeptide;
7) addition of a sequence complementary to the Shine-Dalgarno sequence before the Shine-Dalgarno sequence of the gene encoding the polypeptide, so as to form a secondary structure that makes the attachment of ribosomes impossible;
8) addition of a promoter, which is to be reverse-transcribed, to the 3' end of the open reading frame (ORF) of the gene sequence encoding the polypeptide (reverse transcription engineering, RTE); or
9) a combination of two or more selected from items 1) to 8), but is not particularly limited thereto.

For example, these are described as follows.

The deletion of a part or the entirety of the gene encoding the polypeptide in item 1) may be the elimination of the entirety of a polynucleotide encoding an endogenous target protein in the chromosome, the replacement with a polynucleotide with deletion of some nucleotides, or the replacement with a marker gene.

The modification of an expression control region (or expression control sequence) in item 2) may be the occurrence of mutation on the expression control region (or expression control sequence) through deletion, insertion, non-conservative or conservative substitution, or a combination thereof, or the replacement with a sequence having weaker activity. The expression control region includes a promoter, an operator sequence, a sequence for encoding a ribosomal binding site, and a sequence for controlling the termination of transcription and translation, but is not limited thereto.

The modification of a nucleotide sequence encoding a start codon or 3'-UTR region of the gene transcript encoding the polypeptide in item 5) may be, for example, the substitution with a nucleotide sequence encoding, rather than an endogenous initiation codon, another initiation codon having a higher expression rate of the polypeptide, but is not limited thereto.

The modification of the amino acid sequence or the polynucleotide sequence in items 4) and 5) may be the occurrence of modification on the sequence through deletion, insertion, non-conservative or conservative substitution, or a combination thereof in the amino acid sequence of the polypeptide or the polynucleotide sequence encoding the polypeptide, or the replacement with an amino acid sequence or polynucleotide sequence modified to have weaker activity or an amino acid sequence or polynucleotide sequence modified to have little activity, so as to weak activity of the polypeptide, but is not limited thereto. For example, the expression of the gene may be inhibited or weakened by introducing mutation into the polynucleotide sequence to form a termination codon.

The introduction of an antisense oligonucleotide (e.g., antisense RNA) complementarily binding to the gene transcript encoding the polypeptide in item 6) may be referred to, for example, the literature [Weintraub, H. et al., Antisense-RNA as a molecular tool for genetic analysis, Reviews - Trends in Genetics, Vol. 1(1) 1986].

The addition of a sequence complementary to the Shine-Dalgarno sequence of the gene encoding the polypeptide before the Shine-Dalgarno sequence so as to form a secondary structure that makes the attachment of ribosomes impossible in item 7) may be making mRNA translation impossible or reducing the translation rate thereof.

The addition of a promoter, which is to be reverse-transcribed, to the 3' end of the open reading frame (ORF) of the gene sequence encoding the polypeptide (reverse transcription engineering, RTE) in item 8) may be making an antisense nucleotide complementary to the gene transcript encoding the polypeptide to thereby weaken the activity of the polypeptide.

The microorganism producing putrescine may be any microorganism that can produce putrescine by means of the introduction of the activity of N-acetyltransferase derived from a strain of the genus *Corynebacterium* and the activity of argE derived from E. *coli* by the above-described method. For the purpose of the present disclosure, the microorganism producing putrescine is a microorganism having increased ability to produce target putrescine, in which the activity of N-acetyltransferase derived from a strain of the genus *Corynebacterium* and the activity of argE derived from *E*. *coli* are introduced by the above-described method and the activity of argJ derived from a strain of the genus *Corynebacterium* is weakened, and the microorganism may be a genetically modified microorganism or a recombinant microorganism, but is not limited thereto. In the present disclosure, the "microorganism producing putrescine" may be used interchangeably with "putrescine-producing microorganism" or "microorganism having putrescine producing ability".

Examples of the microorganism may include microorganisms belonging to the genus *Corynebacterium,* the genus *Escherichia,* the genus *Enterbacter,* the genus *Erwinia,* the genus *Serratia,* the genus *Providencia, and the genus Brevibacterium* and, specifically, may be a microorganism of the genus *Corynebacterium.*

More specifically, the microorganism of the genus *Corynebacterium* may be *Corynebacterium glutamicum, Corynebacterium ammoniagenes, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium pollutisoli, Cxorynebacterium imitans, Corynebacterium testudinoris, Corynebacterium flavescens,* or the like, and may be *Corynebacterium glutamicum,* and any microorganism belonging to the genus *Corynebacterium* may be included without limitation.

In accordance with another aspect of the present disclosure, there is provided a method for producing putrescine, the method including culturing, in a medium, a microorganism of the genus *Corynebacterium* having putrescine producing ability, into which activity of an N-acetyltransferase derived from a strain of the genus *Corynebacterium* and activity of an acetylornithine deacetylase (argE) derived from *E. coli* are introduced. The N-acetyltransferase, acetylornithine deacetylase, putrescine, and microorganism are as described above.

The microorganism of the genus *Corynebacterium* may have weakened activity of bifunctional ornithine acetyltransferase/N-acetylglutamate synthase (argJ) derived from a strain of the genus *Corynebacterium,* but is not limited thereto.

The method can be easily determined by a person skilled in the art under optimized culturing conditions and enzyme activity conditions known in the art. Specifically, the step of culturing the microorganism is not particularly limited, but the step may be performed by a known batch culture method, continuous culture method, fed-batch culture method, or the like. The conditions for culturing may not be particularly limited, but the adjustment to appropriate pH (e.g., pH 5 to pH 9, specifically pH 6 to pH 8, and most specifically pH 6.8) may be attained using a basic compound (e.g., sodium hydroxide, potassium hydroxide, or ammonia) or an acidic compound (e.g., phosphoric acid or sulfuric acid), and an aerobic condition may be maintained by introducing oxygen or an oxygen-containing gas mixture to the culture. The temperature for culturing may be maintained at 20 to 45°C, and specifically 25 to 40°C, and the culturing may be performed for about 10 to 160 hours, but are not limited thereto. Putrescine produced by culturing may be released into the medium or may remain in cells.

Furthermore, in the medium for culturing to be used, as a carbon source, sugars and carbohydrates (e.g., glucose, sucrose, lactose, fructose, maltose, molasses, starch, and cellulose), oils and fats (e.g., soybean oil, sunflower seed oil, peanut oil, and coconut oil), fatty acids (e.g., palmitic acid, stearic acid, and linoleic acid), alcohols (e.g., glycerol and ethanol), organic acids (e.g., acetic acid), and the like may be used alone or in combination, but the carbon source is not limited thereto. As a nitrogen source, nitrogen-containing organic compounds (e.g., peptone, yeast extract, meat extract, malt extract, corn steep liquor, soybean flour, and urea) or inorganic compounds (e.g., ammonium sulfate, ammonium chloride, ammonium phosphate, ammonium carbonate, and ammonium nitrate), and the like may be used alone or in combination, but the nitrogen source is not limited thereto. As a phosphorus source, potassium dihydrogen phosphate, dipotassium hydrogen phosphate, a sodium-containing salt corresponding thereto, and the like may be used alone or in a mixture, but the phosphorus sources are not limited thereto. Additionally, the medium may also contain other metal salts (e.g., magnesium sulfate or iron sulfate) and essential growth-promoting substances, such as amino acids and vitamins.

The method may further include recovering putrescine from the cultured medium or microorganism after the culturing step, but is not limited thereto.

As for a method for recovering putrescine produced in the culturing step, target putrescine may be recovered from the culture by using an appropriate method known in the art according to the culturing method. For example, centrifugation, filtration, anion exchange chromatography, crystallization, HPLC, and or like may be used, and target putrescine can be recovering from the medium or microorganism by using an appropriate method known in the art.

The method for recovering putrescine may further include a purifying step. The purifying step may be performed by using an appropriate method known in the art. Therefore, the recovering putrescine may have a purified form or may be a microorganism fermented liquid containing putrescine.

In accordance with still another aspect of the present disclosure, there is provided a composition for producing putrescine, the composition containing a microorganism of the genus *Corynebacterium* having putrescine producing ability, into which activity of an N-acetyltransferase derived from a strain of the genus *Corynebacterium* and activity of an acetylornithine deacetylase (argE) derived from *E. coli* are introduced.

The N-acetyltransferase, acetylornithine deacetylase, putrescine, and microorganism are as described above.

The microorganism of the genus *Corynebacterium* may have weakened activity of bifunctional ornithine acetyltransferase/N-acetylglutamate synthase (argJ) derived from a strain of the genus *Corynebacterium,* but is not limited thereto.

The composition for producing putrescine may include, without limitation, an element capable of introducing activity of an N-acetyltransferase derived from a strain of the genus *Corynebacterium* and activity of an acetylornithine deacetylase (argE) derived from *E. coli.* Specifically, the element may be in the form of being contained in a vector so as to express a gene operably linked to a host cell in which the element is introduced, and the form is as described above.

The N-acetyltransferase derived from the strain of the genus *Corynebacterium* may contain the amino acid sequence of SEQ ID NO: 1 or an amino acid sequence having sequence identity of at least 90% thereto, and this is described above.

The N-acetyltransferase derived from the strain of the genus *Corynebacterium* may contain the amino acid sequence of SEQ ID NO: 1 or an amino acid sequence having sequence identity of at least 90% thereto, and this is described above.

The acetylornithine deacetylase derived from *E. coli* may contain the amino acid sequence of SEQ ID NO: 3 or an amino acid sequence having sequence identity of at least 90% thereto, and this is described above.

The composition of the present disclosure may further contain any appropriate excipient that is usually used in compositions for producing amino acids, and examples of the excipient may be a preserving agent, a wetting agent, a dispersing agent, a suspending agent, a buffer, a stabilizing agent, or an isotonic agent, but are not limited thereto.

In accordance with still another aspect of the present disclosure, there is provided use of a composition for the production of putrescine.

In accordance with still another aspect of the present disclosure, there is provided use of a microorganism of the genus *Corynebacterium* having putrescine producing ability for the production of putrescine, wherein activity of an N-acetyltransferase derived from a strain of the genus *Corynebacterium* and activity of an acetylornithine deacetylase (argE) derived from *E*. *coli* are introduced into the microorganism.

### [Mode for Carrying Out the Invention]

Hereinafter, the present disclosure will be described in detail with reference to exemplary embodiments. However, it would be obvious to a person skilled in the art that these exemplary embodiments are provided for the purpose of illustration only and are not intended to limit the scope of the present disclosure.

### Example 1: Putrescine producing ability of Coryne-derived argJ gene-enhanced strain

### 1-1. Preparation of strain having ATCC13869-derived argJ enhanced in transposon gene of ATCC13032-based putrescine producing strain

To enhance argJ gene encoding bifunctional ornithine acetyltransferase/N-acetylglutamate synthase derived from a strain of the genus *Corynebacterium* in the *Corynebacterium glutamicum* ATCC13032-based putrescine-producing strain, the argJ gene was introduced into the transposon gene of the strain.

As a transformation vector enabling the introduction of a gene into the chromosome by using a transposon gene region of a microorganism of the genus *Corynebacterium,* pDZTn (WO 2009/125992) was used. In addition, and as a promoter, lysCP1 promoter (WO 2009/096689, SEQ ID NO: 9) obtained by modifying a promoter of lysC-asd operon gene derived from *Corynebacterium glutamicum* was used.

Specifically, the pDZTn vector was constructed as follows. To obtain the transposon gene, the nucleotide sequence information about the transposon gene (NCBI accession NO. NC_003450, NCgl1021) of the full nucleotide sequence originate from *Corynebacterium glutamicum* ATCC13032 was obtained from NIH GenBank, and on the basis of the information, two pairs of primers (Table 1, SEQ ID. NOS: 10 to 13) were synthesized.

PCR was performed using the chromosomal DNA of *Corynebacterium glutamicum* ATCC13032 as a template along with the primer pair of SEQ ID NOS: 10 and 13. PfuUltra^{™} High-Fidelity DNA Polymerase (Stratagene) was used as a polymerase, and PCR conditions were set to 30 cycles of denaturing at 96°C for 30 sec, annealing at 58°C for 30 sec, and extending at 72°C for 1 min.

**[TABLE 1]**

| SEQ ID NO | Name | Sequence |
|---|---|---|
| 10 | Tn-A-F | atcctctagagtcgaccatcgctgacaccatctgcc |
| 11 | Tn-A-R | gggcccactagtctcgagttcaccgcgggagccaagcc |
| 12 | Tn-B-F | ctcgagactagtgggccctggattccaaggctacgcc |
| 13 | Tn-B-R | atgcctgcaggtcgaccctgaatggataaggcaccg |

As a result, two pairs of transposon genes (Tn-A, Tn-B) including promoter regions of about 500 bp were obtained as PCR products. Tn-A (SEQ ID NO: 14) was amplified using SEQ ID NOS: 10 and 11 as primers, and Tn-B (SEQ ID NO: 15) was amplified using SEQ ID NOS: 12 and 13 as primers. The amplification products were cloned into the pDZ vector previously treated with Sall restriction enzyme by using a BD In-Fusion kit (BD), thereby obtaining pDZTn vector. A plurality of restriction enzyme recognition sites artificially inserted during primer construction were included between the two amplified products.

The lysCP1 promoter was prepared as follows. PCR was performed using, as a template, the chromosomal DNA of CA01-0135 strain (WO 2009-096689, KCCM10919P) obtained by transforming *Corynebacterium glutamicum* KFCC10881 with a vector containing the lysCP1 promoter, along with the primers of SEQ ID NOS: 16 and 17 below. PfuUltra^{™} High-Fidelity DNA Polymerase (Stratagene) was used as a polymerase, and PCR conditions were set to 30 cycles of denaturing at 95°C for 30 sec, annealing at 55°C for 30 sec, and extending at 72°C for 30 sec.

The sequences of the primers used are shown in Table 2 below.

**[TABLE 2]**

| SEQ ID NO | Name | Sequence |
|---|---|---|
| 16 | lysCP1 promotor_F | GAATGAGTTCCTCGAGCCGATGCTAGGGCGAA AA |
| 17 | lysCP1 promotor_R | CTTTGTGCACCTTTCGATCTACGTGCTGACAGT TAC |

As a result, the lysCP1 promoter region (SEQ ID NO: 9) was obtained as a PCR product.

To obtain the argJ gene, the primer pair of SEQ ID NOS: 18 and 19 for obtaining argJ open-reading frame (ORF) homologous recombinant fragments on the basis of the nucleotide sequence (SEQ ID NO: 6) of *Corynebacterium glutamicum* ATCC13869-derived argJ gene was prepared.

PCR was performed using the chromosomal DNA of *Corynebacterium glutamicum* ATCC13869 as a template along with the primer pair of SEQ ID NOS: 18 and 19. PfuUltra^{™} High-Fidelity DNA Polymerase (Stratagene) was used as a polymerase, and PCR conditions were set to 30 cycles of denaturing at 95°C for 30 sec, annealing at 55°C for 30 sec, and extending at 72°C for 1 min and 30 sec.

The sequences of the primers used are shown in Table 3 below.

**[TABLE 3]**

| SEQ ID NO | Name | Sequence |
|---|---|---|
| 18 | argJ_F | GTTCCACATGGCCAAAAAAGGCATTAC |
| 19 | argJ_R | TTCTTTTTAAGAGCTGTACGCGGAGTTGATCTCC |

As a result, argJ gene fragments of about 1.2 kb were amplified as PCR products. The fragments were electrophoresed on a 0.8% agarose gel, and bands with desired sizes were eluted and purified.

The previously prepared pDZTn vector was treated with the restriction enzyme Xhol, and the respective PCR products (lysCP1 promoter region and argJ gene fragments) obtained above were fusion-cloned into the vector by using the In-Fusion^{®} HD Cloning Kit (Clontech), and then the resulting plasmid was named pDZTn-lysCP1-argJ.

Then, the constructed plasmid pDZTn-lysCP1-argJ was introduced into CC01-0163 strain (US 9677099, KCCM11240P) by electroporation to obtain a transformant, wherein the strain was obtained by deletion of activity of NCgl1469 protein, defined as histone acetyltransferase HPA2 and related acetyltransferase in CC01-0064 strain (US 9890404, KCCM11138P) having putrescine producing ability, which was obtained by deletion of the gene encoding ornithine carbamoyl transferase (argF) and the gene (NCgl1221) encoding the glutamate exporter involved in glutamate export, introduction of the gene (speC) encoding ornithine decarboxylase (ODC) derived from wild-type E. *coli* 3110 strain into the chromosome, and replacement of the promoter of ArgCJBD gene cluster encoding enzymes involved in the synthesis of ornithine from glutamate in the wild-type *Corynebacterium glutamicum* ATCC13032. The transformant was plated and cultured on BHIS plate medium (Braine heart infusion 37 g/l, sorbitol 91 g/l, and agar 2%) containing 25 µg/ml kanamycin and X-gal (5-bromo-4-chloro-3-indolin-D-galactoside) to form colonies. By selecting white colonies from the colonies thus formed, transformant strains into which the plasmid pDZTn-lysCP1-argJ was introduced were selected.

The selected strains were cultured with shaking in CM medium (glucose (10 g/L), polypeptone (10 g/L), yeast extract (5 g/L), beef extract (5 g/L), NaCl (2.5 g/L), urea (2 g/L), and pH 6.8) at 30°C for 8 hours. Subsequently, each cell culture was serially diluted from 10⁻⁴ to 10⁻¹⁰ and then plated and cultured on an X-gal-containing solid medium to form colonies. By selecting white colonies appearing at a relatively low frequency among the colonies formed, a strain in which the gene encoding argJ was introduced was finally selected by second crossover.

PCR was performed on the finally selected strain by using the primer pair of SEQ ID NOS: 18 and 19 to confirm that the gene encoding argJ was introduced, and the *Corynebacterium glutamicum* mutant strain was named CC01-0163 Tn:lysCP1-argJ.

### 1-2. Assessment of putrescine producing ability of Coryne-derived argJ gene-enhanced Coryne-based strain

To investigate the effect on putrescine production when the argJ gene derived from *Corynebacterium* was enhanced in a putrescine-producing strain, a comparison of putrescine production ability was conducted on the *Corynebacterium glutamicum* mutant strain prepared in Example 1-1.

Specifically, the *Corynebacterium glutamicum* mutant strain (CC01-0163 Tn:lysCP1-argJ) prepared in Example 1-1 was plated on CM plate medium (1% glucose, 1% polypeptone, 0.5% yeast extract, 0.5% beef extract, 0.25% NaCl, 0.2% urea, 100 µl of 50% NaOH, 2% agar, pH 6.8, based on 1 L) containing 1 mM arginine, and cultured at 30°C for 24 hours.

About one platinum loop of each strain thus cultured was inoculated in 25 ml of a titer medium (8% glucose, 0.25% soybean protein, 0.50% corn steep solids, 4% (NH₄)₂SO₄, 0.1% KH₂PO₄, 0.05% MgSO₄·7H₂O, 0.15% urea, 100 µg of biotin, 3 mg of thiamine hydrochloride, 3 mg of calcium-pantothenic acid, 3 mg of nicotinamide, 5% CaCOs, based on 1 L), and then cultured with shaking at 30°C and 200 rpm for 98 hours. Then, 1 mM arginine was added to the medium for culturing each strain. The concentration of putrescine produced from each culture was measured, and the results are shown in Table 4 below.

**[TABLE 4]**

| Strain name | Putrescine (g/L) |
|---|---|
| CC01-0163 | 11.7 |
| CC01-0163 Tn:lysCP1-argJ | 12.4 |

As a result, as shown in Table 4, the putrescin production was increased by 6% in the *Corynebacterium glutamicum* mutant strain in which *Corynebacterium-derived* argJ was enhanced.

### Example 2: Introduction of E. coli-derived arga and argE into putrescine-producing strain and putrescine producing ability thereof

### 2-1. Preparation of strain by co-introduction of both E. coli-derived argA and argE into transposon gene of ATCC13032-based putrescine-producing strain

To investigate whether putrescine producing ability was improved when argA encoding *E. coli*-derived N-acetylglutamate synthase and argE encoding *E*. *coli*-derived acetylornithine deacetylase were inserted into *Corynebacterium glutamicum* ATCC13032-based putrescine-producing strain (CC01-0163), argA and argE were introduced into the transposon gene of the strain.

To this end, the primer pair of SEQ ID NOS: 21 and 22 for obtaining homologous recombinant fragments in the argA ORF region from the nucleotide sequence (SEQ ID NO: 20) of the *E. coli*-derived argA gene was prepared, and the primer pair of SEQ ID NOS: 23 and 24 for obtaining homologous recombinant fragments in the argE ORF region from the nucleotide sequence (SEQ ID NO: 4) of the argE gene was prepared.

The sequences of the primers are shown in Table 5.

**[TABLE 5]**

| SEQ ID NO | Name | Sequence |
|---|---|---|
| 21 | argA ORF_F | GAAAGGTGCACAAAGATGGTAAAGGAACGTAAAACCG |
| 22 | argA ORF_R | GCCCACTAGTCTCGAGCATGCGGCGTTGATTTTG |
| 23 | argE ORF_F | GAAAGGTGCACAAAGATGAAAAACAAATTACCGCC |
| 24 | argE ORF_R | GCCCACTAGTCTCGAGGTTTGAGTCACTGTCGGTCG |

The lysCP1 promoter region was obtained through PCR using the chromosome of KCCM10919P strain as a template along with the primer pair of SEQ ID NOS: 16 and 17 by the same method as in Example 1-1.

To obtain argA gene, PCR was performed by the chromosome of *E. coli* W3110 strain as a template along with the primer pair of SEQ ID NOS: 21 and 22. PfuUltra^{™} High-Fidelity DNA Polymerase (Stratagene) was used as a polymerase, and PCR conditions were set to 30 cycles of denaturing at 95°C for 30 sec, annealing at 55°C for 30 sec, and extending at 72°C for 1 min 30 sec.

As a result, argA gene fragments of about 1.6 kb were amplified as PCR products, and the fragments were fusion-cloned, together with the lysCP1 promoter region, into the pDZTn vector prepared in Example 1-1 by the same method as in Example 1-1. The resulting plasmid was named pDZTn-lysCP1-argA.

The pDZTn vector prepared in Example 1-1 was treated with the restriction enzyme Xhol, and the respective PCR products (lysCP1 promoter region and argA gene fragment) obtained above were fusion-cloned into the vector by the same method as in Example 1-1, and then the resulting plasmid was named pDZTn-lysCP1-argA.

To obtain argE gene, PCR was performed by the chromosome of E. *coli* W3110 strain as a template along with the primer pair of SEQ ID NOS: 23 and 24 by the same method as above. PfuUltra^{™} High-Fidelity DNA Polymerase (Stratagene) was used as a polymerase, and PCR conditions were set to 30 cycles of denaturing at 95°C for 30 sec, annealing at 55°C for 30 sec, and extending at 72°C for 1 min 30 sec.

As a result, argE gene fragments of about 1.4 kb were amplified as PCR products, and the fragments were fusion-cloned, together with the previously obtained lysCP1 promoter region, into the pDZTn vector prepared in Example 1-1 by the same method as in Example 1-1. The resulting plasmid was named pDZTn-lysCP1 -arge.

Then, the plasmid pDZTn-lysCP1-argA was introduced into CC01-0163 by electrophoresis to obtain transformant strains, and transformant strains in which the plasmid pDZTn-lysCP1-argA was introduced were selected by the same method as in Example 1-1.

The selected strains were subjected to second crossover to finally select a strain in which the gene encoding argA was introduced. PCR was performed on the finally selected strain by using the primer pair of SEQ ID NOS: 21 and 22 to confirm that the gene encoding argA was introduced, and the *Corynebacterium glutamicum* mutant strain was named CC01-0163 Tn:lysCP1-argA.

To introduce argE into the prepared *Corynebacterium glutamicum* mutant strain into which argA was introduced, the previously constructed pDZTn-lysCP1-argE was transformed into CC01-0163 Tn:lysCP1-argA by the same method as above, and PCR was performed on the finally selected strain along with the primer pair of SEQ ID NOs: 23 and 24 to confirm that argE was introduced into the transposon.

The *Corynebacterium glutamicum* mutant strain thus selected was called CC01 -0163 Tn:lysCP1-argA Tn:lysCP1-argE.

### 2-2. Assessment of putrescine producing ability of putrescine-producing strain of the genus Corynebacterium in which E. coli-derived argA and argE were introduced

To investigate the effect, on putrescine production, of the introduction of *E. coli*-derived argA and argE into a putrescine-producing strain, a comparison of putrescine production ability was conducted on the *Corynebacterium glutamicum* mutant strain prepared in Example 2-1.

The *Corynebacterium glutamicum* mutant strain (CC01-0163 Tn:lysCP1-argA Tn:lysCP1-argE) prepared in Example 2-1 and the parent strain (CC01-0163) were each cultured by the same method as in Example 1-2, and the concentration of putrescine produced from each culture was measured. The results are shown in Table 6 below.

**[TABLE 6]**

| Strain name | Putrescine (g/L) |
|---|---|
| CC01-0163 | 12.2 |
| CC01 -0163 Tn:lysCP1-arga Tn:lysCP1-argE | 13.4 |

As a results, as shown in Table 6, the putrescine production was increased by 9.8% in the *Corynebacterium glutamicum* mutant strain in which *E. coli*-derived argA and argE genes were introduced.

### Example 3: Putrescine producing ability of Coryne-derived Ncgl2644 gene-enhanced strain

### 3-1. Preparation of strain in which ATCC13869-derived Ncgl2644 was introduced into transposon gene of ATCC13032-based putrescine producing strain

To investigate whether the putrescine producing ability was improved when Ncgl2644 gene encoding *Corynebacterium glutamicum* ATCC13869-derived GCN5-related N-acetyltransferases (GNAT) family N-acetyltransferase was enhanced in *Corynebacterium glutamicum* ATCC13032-based putrescine-producing strain (CC01-0163), Ncgl2644 was introduced and enhanced in the transposon gene of the strain.

To this end, the primer pair of SEQ ID NOS: 25 and 26 for obtaining homologous recombinant fragments of the Ncgl2644 ORF region from the nucleotide sequence (SEQ ID NO: 2) of the *Corynebacterium-derived* gene Ncgl2644 was prepared.

The sequences of the primers are shown in Table 7.

**[TABLE 7]**

| SEQ ID NO | Name | Sequence |
|---|---|---|
| 25 | Ncgl2644 ORF_F | GAGGAGATCAAAACACATATGACGCCTAGTCTTCCCCG |
| 26 | Ncgl2644 ORF_R | TTAGAATTTCCGTTCGGCGTACC |

To obtain Ncgl2644 gene, PCR was performed by the chromosome of the Corynebacterium glutamicum ATCC13869 strain as a template along with the primer pair of SEQ ID NOS: 25 and 26. PfuUltra^{™} High-Fidelity DNA Polymerase (Stratagene) was used as a polymerase, and PCR conditions were set to 30 cycles of denaturing at 95°C for 30 sec, annealing at 55°C for 30 sec, and extending at 72°C for 1 min. As a result, Ncgl2644 gene fragments of about 1 kb were amplified as PCR products, and the fragments were fusion-cloned, together with the lysCP1 promoter region obtained in Example 2-1, into the pDZTn vector prepared in Example 1-1 by the same method as in Example 1-1. The resulting plasmid was named pDZTn-lysCP1-Ncgl2644.

Then, the plasmid pDZTn-lysCP1-Ncgl2644 was introduced into CC01-0163 by electrophoresis to obtain transformant strains, and transformant strains in which the plasmid pDZTn-lysCP1- Ncgl2644 was introduced were selected by the same method as in Example 1-1.

The selected strains were subjected to second crossover to finally select a strain in which the gene encoding Ncgl2644 was introduced. PCR was performed on the finally selected strain by using the primer pair of SEQ ID NOS: 25 and 26 to confirm that the gene encoding Ncgl2644 was introduced, and the *Corynebacterium glutamicum* mutant strain was named CC01-0163 Tn:lysCP1-Ncgl2644.

### 3-2. Assessment of putrescine producing ability of putrescine-producing strain of genus Corynebacterium in which Coryne-derived Ncgl2644 was enhanced

To investigate the effect on putrescine production when the Ncgl2644 gene derived from Corynebacterium was enhanced in a putrescine-producing strain, a comparison of putrescine production ability was conducted on the Corynebacterium glutamicum mutant strain prepared in Example 3-1.

Specifically, the Corynebacterium glutamicum mutant strain (CC01-0163) prepared in Example 3-1 and the parent strain (CC01-0163) were each cultured by the same method as in Example 1-2, and the concentration of putrescine produced from each culture was measured. The results are shown in Table 8 below.

**[TABLE 8]**

| Strain name | Putrescine (g/L) |
|---|---|
| CC01-0163 | 12.0 |
| CC01-0163 Tn:lysCP1-Ncgl2644 | 10.1 |

As a result, as shown in Table 8, the putrescine producing ability was reduced when *Corynebacterium*-derived Ncgl2644 was enhanced. This may result from the occurrence of a bottleneck in the bio-synthetic pathway caused by increase in the concentration of acetyl glutamate, an intermediate metabolite in the putrescine production pathway. Therefore, the effect of Ncgl2644 on putrescine production was assessed through additional examples below.

### Example 4: Putrescine producing ability of argJ gene-deleted and Ncgl2644-enhanced strain

### 4-1. Preparation of strain in which araJ was deleted in ATCC13032-based putrescine-producing strain

A strain in which *Corynebacterium glutamicum* ATCC13032-derived argJ gene was deleted in *Corynebacterium glutamicum* ATCC13032-based putrescine-producing strain (CC01-0163) was prepared.

To this end, the primer pair of SEQ ID NOS: 27 and 28 for obtaining homologous recombinant fragments of the N-terminus region of argJ and the primer pair of SEQ ID NOS: 29 and 30 for obtaining homologous recombinant fragments of the C-terminus region of argJ from the nucleotide sequence (SEQ ID NO: 8) of the *Corynebacterium glutamicum* ATCC13032-derived argJ gene were prepared.

The sequences of the primers are shown in Table 9.

**[TABLE 9]**

| SEQ ID NO | Name | Sequence |
|---|---|---|
| 27 | argJ_N_F | CGGGATCCCACGCCTGTCTGGTCGC |
| 28 | argJ_N_R | ACGCGTCGACGGATCTAAAGCGGCGCTTC |
| 29 | argJ_C_F | ACGCGTCGACTCCGGCGCTGACATTGATGTCC |
| 30 | argJ_C_R | CTAGTCTAGAGAGCTGCACCAGGTAGACG |

To obtain homologous recombinant fragments of the C-terminus region and the N-terminus region of argJ, PCR was performed using the genomic DNA of *Corynebacterium glutamicum* ATCC13032 as a template along with the primer pair of SEQ ID NOS: 27 and 28 and the primer pair of SEQ ID NOS: 29 and 30. PfuUltra^{™} High-Fidelity DNA Polymerase (Stratagene) was used as a polymerase, and PCR conditions were set to 30 cycles of denaturing at 95°C for 30 sec, annealing at 55°C for 30 sec, and extending at 72°C for 30 sec. As a result, PCR fragments of the N-terminal region and the C-terminal region were amplified as PCR products, respectively, and the fragments were electrophoresed on a 0.8% agarose gel, and bands with desired sizes were eluted and purified.

The obtained fragments of the N-terminus region were treated with the restriction enzymes BamHI and Sall and the obtained fragments of the C-terminus region were treated with the restriction enzymes Sall and Xbal. The treated fragments were cloned into pDZ vector treated with the restriction enzymes BamHI and Xbal to construct pDZ-1'argJ(K/O) plasmid.

The constructed plasmid pDZ-1'argJ(K/O) was introduced into *Corynebacterium glutamicum* CC01-0163 by electrophoresis to obtain a transformant, and pDZ-1'NCglargJ(K/O)-introduced strains were selected by the same method as in Example 1-1.

A Corynebacterium glutamicum strain having weakened putrescine productivity by deletion of the gene encoding argJ was finally selected from the selected strains, and the Corynebacterium glutamicum mutant strain with weakened putrescine exporting ability was named CC01-0163 ΔargJ.

Ncgl2644 gene was transformed into the prepared CC01-0163 ΔargJ strain by the same method as in Example 3-1 to prepare CC01-0163 ΔargJ Tn:lysCP1-Ncgl2644 strain.

### 4-2. Assessment of putrescine producing ability of putrescine-producing strain of genus Corynebacterium in which Coryne-derived argJ was deleted and Ncgl2644 was enhanced

To investigate the effect on putrescine production when the Corynebacterium-derived argJ gene was deleted and the Corynebacterium-derived Ncgl2644 gene was enhanced in a putrescine-producing strain, a comparison of putrescine production ability was conducted on the Corynebacterium glutamicum mutant strain prepared in Example 4-1.

Specifically, the Corynebacterium glutamicum mutant strain (CC01-0163 △argJ Tn:lysCP1-Ncgl2644) and the parent strain (CC01-0163 ΔargJ) were each cultured by the same method as in Example 1-2, and the concentration of putrescine produced from each culture was measured. The results are shown in Table 10 below.

**[TABLE 10]**

| Strain name | Putrescine (g/L) |
|---|---|
| CC01-0163 ΔargJ | 0.1 |
| CC01-0163 ΔargJ Tn:lysCP1-Ncgl2644 | 0.3 |

As a result, as shown in Table 10, the argJ-deleted strain had difficulty in having putrescine producing ability, and even though Ncgl2644 capable of generating acetyl glutamate was introduced, the production of putrescin was low due to the absence of an enzyme that can deacetylate acetyl ornithine, an intermediate metabolite, generated after the introduction. Therefore, a Ncgl2644 and argE co-enhanced strain was assessed for putrescine producing ability through additional working examples.

### Example 5: Putrescine producing ability of Coryne-derived Ncgl2644 gene-enhanced and E coli-derived argE gene-introduced strains

### 5-1. Preparation of Ncgl2644 gene-enhanced strains from ATCC13032-based putrescine producing strains

Into the CC01-0163 Tn:lysCP1-Ncgl2644 strain prepared in Example 3-1 and the CC01-0163 ΔargJ Tn:lysCP1-Ncgl2644 strain prepared in Example 4-1, argE encoding *E*. *coli* W3110-derived acetylornithine deacetylase capable of deacetylating acetyl ornithine was introduced.

Specifically, the pDZTn-lysCP1-argE plasmid constructed in Example 2-1 was transformed into the CC01-0163 Tn:lysCP1-Ncgl2644 strain and the CC01-0163 ΔargJ Tn:lysCP1-Ncgl2644 strain by the same method as in Example 2-1 to obtain transformants, and transformant strains into which the pDZTn-lysCP1-argE plasmid was introduced were selected by the same method as in Example 1-1.

The argE-introduced *Corynebacterium glutamicum* strains were finally selected from the selected strains, and the prepared *Corynebacterium glutamicum* strains CC01-0163 Tn:lysCP1-Ncgl2644 Tn:lysCP1-argE and CC01-0163 ΔargJ Tn:lysCP1-Ncgl2644 Tn:lysCP1-argE were prepared. The CC01-0163 ΔargJ Tn:lysCP1-Ncgl2644 Tn:lysCP1-argE was named *Corynebacterium glutamicum* CC01-1425. The *Corynebacterium glutamicum* CC01-1425 (CC01-0163 ΔargJ Tn:lysCP1-Ncgl2644 Tn:lysCP1-argE) was deposited (KCCM12774P) on 24 July 2020.

### 5-2. Assessment of putrescine producing ability of putrescine-producing strain of genus Corynebacterium in which Ncgl2644 gene and argE were enhanced

To investigate the effect on putrescine production when the Corynebacterium-derived Ncgl2644 was enhanced and Corynebacterium-derived argE was introduced in a putrescine-producing strain, a comparison of putrescine production ability was conducted on the Corynebacterium glutamicum mutant strain prepared in Example 5-1.

Specifically, the Corynebacterium glutamicum mutant strains (CC01-0163 Tn:lysCP1-Ncgl2644 Tn:lysCP1-argE and CC01-0163 △argJ Tn:lysCP1-Ncgl2644 Tn:lysCP1-argE) and the parent strain (CC01-0163) were each cultured by the same method as in Example 1-2, and the concentration of putrescine produced from each culture was measured. The results are shown in Table 11 below.

**[TABLE 11]**

| Strain name | Putrescine (g/L) |
|---|---|
| CC01-0163 | 12.0 |
| CC01-0163 Tn:lysCP1-Ncgl2644 Tn:lysCP1-argE | 15.7 |
| CC01-0163 ΔargJ Tn:lysCP1-Ncgl2644 Tn:lysCP1-argE | 16.4 |

As shown in Table 11, the co-introduction of Ncgl2644 and argE led to significantly improved putrescine production compared with the CC01-0163 strain. Especially, the CC01-0163 ΔargJ Tn:lysCP1-Ncgl2644 Tn:lysCP1-argE strain with argJ activity eliminated showed a putrescin producing ability improved by 36.6% compared with the CC01-0163 strain, and the CC01-0163 Tn:lysCP1-Ncgl2644 Tn:lysCP1-argE strain with argJ activity remaining showed low putrescine producing ability compared with the strain with argJ activity eliminated, but had a putrescin producing ability improved by only 30.8% compared with the CC01-0163 strain. In addition, the CC01-0163 Tn:lysCP1-Ncgl2644 Tn:lysCP1-argE strain with argJ activity remaining showed significantly higher putrescine production compared with the *Corynebacterium glutamicum* mutant strain into which *E. coli-*derived argA and argE exhibiting similar functions to Ncgl2644 were co-introduced (Table 6).

It was confirmed thought the results of the present examples that the deletion of argJ and co-expression of Ncgl2644 and argE led to more remarkable putrescine producing ability compared with the non-deletion of argJ or the deletion of argJ and the introduction of *E. coli*-derived argA and argE exhibiting similar functions to Ncgl2644.

As set forth above, a person skilled in the art to which the present disclosure pertains will be able to understand that the present disclosure may be embodied in other specific forms without departing from the technical spirit or essential characteristics thereof. Therefore, the exemplary embodiments described above should be construed as being exemplified and not limiting the present disclosure. The scope of the present disclosure should be understood that all changes or modifications derived from the definitions and scopes of the claims and their equivalents fall within the scope of the disclosure.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CC01-1425 | Accession number given by the
INTERNATIONAL DEPOSITARY AUTHORITY:
KCCM12774P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on July. 24. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: July. 24. 2020.

¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS [?]
Yoolim Bldg., 45, Hongjenae 2ga-gil, Seodaemun-gu, Seoul, 03641, Korea Tel: 82-2-391-0950, 396-0950 Fax: 82-2-392-2859

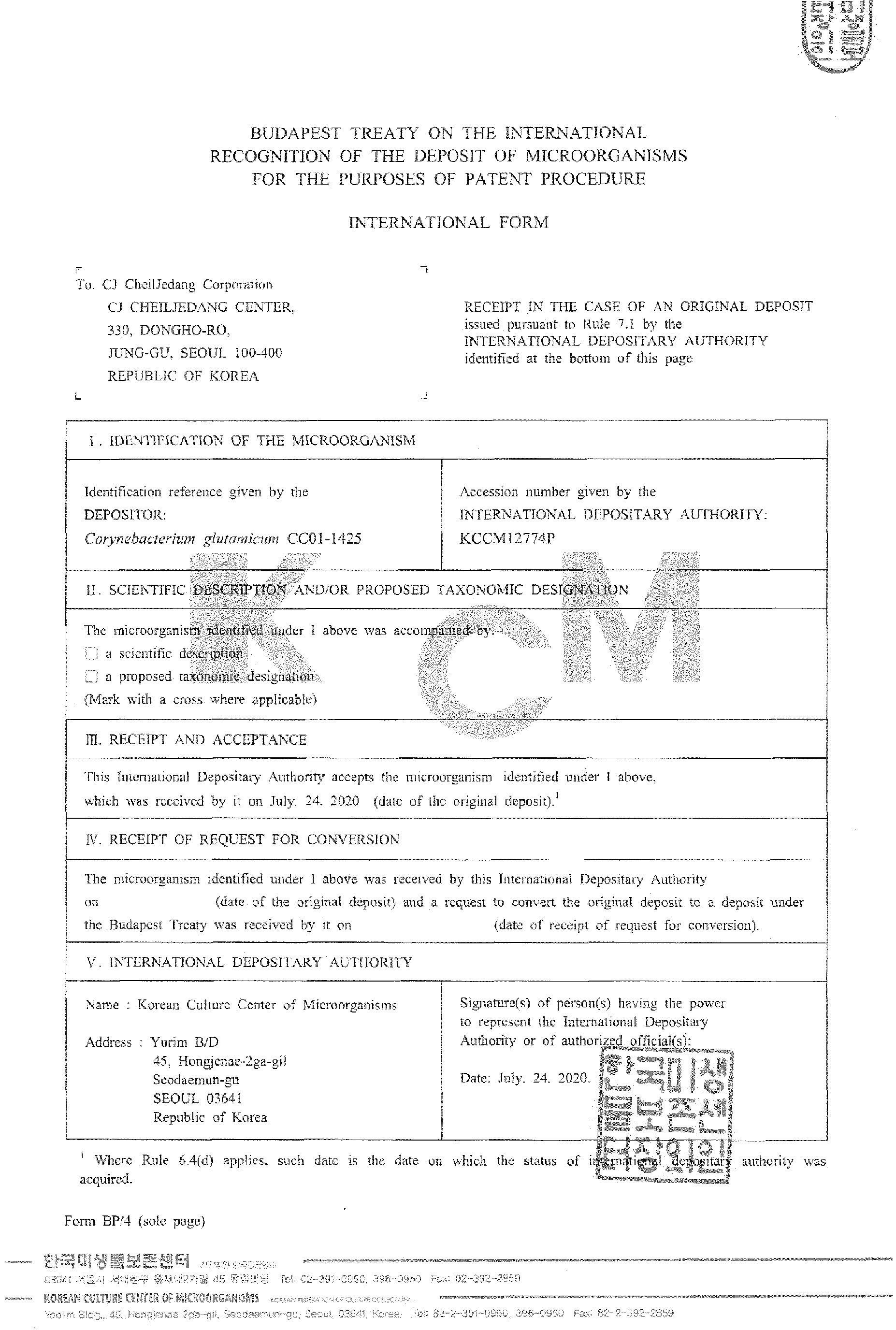
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CC01-1425 | Accession number given by the
INTERNATIONAL DEPOSITARY AUTHORITY:
KCCM12774P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on July. 24. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: July. 24. 2020.

¹ Where Rule 6.4(d) applies, such date is the date on which the status of international depositary authority was
acquired.

Form BP/4 (sole page)

한국미생물보존센터 [?]
03641 서울시 서대문구 홍제내2가길 45 유림빌딩 Tel: 02-391-0950, 396-0950 Fax: 02-392-2859
KOREAN CULTURE CENTER OF MICROORGANISMS [?]
Yoolim Bldg., 45, Hongjenae 2ga-gil, Seodaemun-gu, Seoul, 03641, Korea Tel: 82-2-391-0950, 396-0950 Fax: 82-2-392-2859

## Claims

1. A microorganism of the genus *Corynebacterium* having putrescine producing ability, into which activity of an N-acetyltransferase derived from a strain of the genus *Corynebacterium* and activity of an acetylornithine deacetylase (argE) derived from E. *coli* are introduced.

2. The microorganism of the genus *Corynebacterium* of claim 1, wherein the N-acetyltransferase derived from the strain of the genus *Corynebacterium* comprises the amino acid sequence of SEQ ID NO: 1 or an amino acid sequence having sequence identity of at least 90% thereto.

3. The microorganism of the genus *Corynebacterium* of claim 1, wherein the acetylornithine deacetylase (argE) derived from *E*. *coli* comprises the amino acid sequence of SEQ ID NO: 3 or an amino acid sequence having sequence identity of at least 90% thereto.

4. The microorganism of the genus *Corynebacterium* of claim 1, wherein the microorganism has weakened activity of bifunctional ornithine acetyltransferase/N-acetylglutamate synthase (argJ) derived from a strain of the genus *Corynebacterium.*

5. The microorganism of the genus *Corynebacterium* of claim 1, wherein the microorganism is *Corynebacterium glutamicum.*

6. A method for producing putrescine, the method comprising culturing, in a medium, a microorganism of the genus *Corynebacterium* having putrescine producing ability, into which activity of an N-acetyltransferase derived from a strain of the genus *Corynebacterium* and activity of an acetylornithine deacetylase (argE) derived from *E*. *coli* are introduced.

7. The method of claim 6, further comprising recovering putrescine from the cultured medium or microorganism.

8. The method of claim 6, wherein the N-acetyltransferase derived from the strain of the genus *Corynebacterium* has the amino acid sequence of SEQ ID NO: 1.

9. The method of claim 6, wherein the acetylornithine deacetylase derived from *E. coli* has the amino acid sequence of SEQ ID NO: 3.

10. A composition for producing putrescine, the composition comprising a microorganism of the genus *Corynebacterium* having putrescine producing ability, into which activity of an N-acetyltransferase derived from a strain of the genus *Corynebacterium* and activity of an acetylornithine deacetylase (argE) derived from *E. coli* are introduced.

11. The composition of claim 10, wherein the N-acetyltransferase derived from the strain of the genus *Corynebacterium* comprises the amino acid sequence of SEQ ID NO: 1 or an amino acid sequence having sequence identity of at least 90% thereto.

12. The composition of claim 10, wherein the acetylornithine deacetylase derived from *E. coli* comprises the amino acid sequence of SEQ ID NO: 3 or an amino acid sequence having sequence identity of at least 90% thereto.

13. Use of a microorganism of the genus *Corynebacterium* having putrescine producing ability for the production of putrescine, wherein activity of an N-acetyltransferase derived from a strain of the genus *Corynebacterium* and activity of an acetylornithine deacetylase (argE) derived from *E*. *coli* are introduced into the microorganism.
